# EUROPEAN PATENT SPECIFICATION

(11) **EP 2 038 282 B1**
(45) Date of publication and mention of the grant of the patent: **21.12.2016**
(21) Application number: 07768581.6
(22) Date of filing: 03.07.2007
(51) Int. Cl.: C07D 487/04, A61K 31/522

(54) **SALTS OF PYRROLOPYRIMIDINONE DERIVATIVES AND PROCESS FOR PREPARING THE SAME**
SALZE VON PYRROLOPYRIMIDINONDERIVATEN UND VERFAHREN ZU DEREN HERSTELLUNG
SELS DE DÉRIVÉS DE PYRROLOPYRIMIDINONE ET MÉTHODE D'OBTENTION

(30) Priority: 03.07.2006 KR 20060062040; 03.07.2006 KR 20060062042; 03.07.2006 KR 20060062043; 03.07.2006 KR 20060062046; 03.07.2006 KR 20060062048
(43) Date of publication of application: 25.03.2009
(73) Proprietor: SK Chemicals, Co., Ltd., Suwon-si, Gyeonggi-do 440-300 (KR)
(72) Inventor: KIM, Jae-Sun, Suwon-si Gyeonggi-do 441-880 (KR); KIM, Nam Ho, Seongnam-si Gyeonggi-do 462-150 (KR); LEE, Jin Young, Suwon-si Gyeonggi-do 440-707 (KR); LEE, Nam Kyu, Jangan-gu Suwon-si 440-842 (KR); LEE, Yoon-Jung, Yongin-si Gyeonggi-do 446-951 (KR); JANG, Woo Jae, Gunpo-si Gyeonggi-do 435-826 (KR); YOUN, Won-No, Seoul 151-015 (KR); OH, Joon Gyo, Suwon-si Gyeonggi-do 440-709 (KR); SUNG, Jin-Heung, Bucheon-si Gyeonggi-do 420-709 (KR); UM, Key An, Suwon-si Gyeonggi-do 440-709 (KR)
(74) Representative: WSL Patentanwälte Partnerschaft mbB
(86) International application number: PCT/KR2007/003213
(87) International publication number: WO 2008/004796

(56) References cited:
- WO-A1-2006/018088
- KR-A- 20010 083 637
- US-A1- 2003 171 361
- OH ET AL: "Validation of a HPLC method for the quantification and purity determination of SK3530 in drug substance and tablet", JOURNAL OF PHARMACEUTICAL AND BIOMEDICAL ANALYSIS, NEW YORK, NY, US, vol. 43, no. 3, 2 February 2007 (2007-02-02), pages 1179-1184, XP005870931, ISSN: 0731-7085, DOI: 10.1016/J.JPBA.2006.10.011

## Description

### [Technical Field]

The present invention relates to salts of a pyrrolopyrimidinone derivative, which are effective PDE-5 inhibitors, and a process for preparing them.

### [Background Art]

Korean Patent No. 358083 and international patent application WO2006/018088A1 disclose pyrrolopyrimidinone derivatives having good inhibition activity against PDE-5, a method of its preparation thereof, an intermediate compound used to prepare the same and their use for prevention and treatment of erectile dysfunction, pulmonary arterial hypertension, chronic obstructive pulmonary disease, benign prostatic hypertrophy, lower urinary tract diseases, and hypopigmentary disorders.

Of the pyrrolopyrimidinone derivatives disclosed in Korean Patent No. 358083 and WO2006/018088A1, 5-ethyl-2-{5-[4- (2-hydroxyethyl)piperazin-1-ylsulfonyl]-2-*n-*propoxyphenyl}-7-*n*-propyl-I-3,5-dihydro-4H-pyrrolo[3,2-d]pyrimidin-4-one hereinafter, "SK-3530") represented by the following formula (1) is an excellent selective inhibitor PDE-5 over other PDEs and is under clinical trial for the treatment of erectile dysfunction after passing through the preclinical stage.

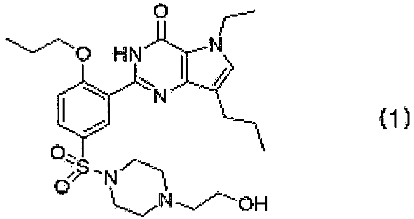

The dihydrochloride salt (2HCl) of SK-3530 has been under investigation through the preclinical and clinical stages.

The SK-3530 dihydrochloride salt has good solubility and can be easily stabilized for pharmaceutical preparation. But, it has the following drawbacks.

First, because the SK-3530 dihydrochloride salt is hygroscopic, it easily absorbs moisture from the atmosphere and becomes discolored when the moisture content is high. And, due to the hygroscopic property, an anhydrous solvent condition and a dry air condition have to be provided to obtain a stable product.

Second, the SK-3530 dihydrochloride salt should be kept at a temperature lower than room temperature because it does not show enough stability at room temperature. In particular, the SK-3530 dihydrochloride salt is labile to heat or light, and thus any prolonged exposure to heat or light results in various impurities.

Third, the SK-3530 dihydrochloride salt could corrode the punch during tablet ting due to its somewhat corrosive properties. This is because the SK-3530 dihydrochloride salt is a simple amorphous salt rather than being a stable crystalline acid addition salt or hydrate form. Thus, one of the two hydrochloric acid groups with a relatively weak ionic bond character may leave the molecule under severe conditions.

As aforementioned, the SK-3530 dihydrochloride salt may be endowed with a
sufficient stability for pharmaceutical preparation. But, some additional techniques and costs are needed due to the deficiency in intrinsic physicochemical property and stability of the compound.

The present inventors have made various research efforts to solve the aforesaid problems of the SK-3530 dihydrochloride salt. In doing so, they discovered that a crystalline acid addition salt of SK-3530 suitable for pharmaceutical preparation is obtained when SK-3530 is prepared into an acid addition salt of gentisate, maleate, citrate, fumarate or hemitartrate instead of hydrochloride.

By reacting a free base of SK-3530 with a pharmaceutically acceptable acid selected from gentisic acid, maleic acid, citric acid, fumaric acid and tartaric acid, the present inventors could prepare new acid salts with sufficient stabilities against temperature, moisture and light. Therefore, they completed the present invention by preparing a novel crystalline acid addition salt of SK-3530, which shows sufficient stabilities and is readily applicable to pharmaceutical preparations.

### [Disclosure of the Invention]

An object of the present invention is to provide a salt of SK-3530 satisfying the physical and chemical requirements needed for a pharmaceutically acceptable salt.

Another object of the present invention is to provide a preparation process of a salt of SK-3530 satisfying the physical and chemical requirements by reacting a free base SK-3530 with a specific acid.

Yet another object of the present invention is to provide a pharmaceutical composition for the treatment and prevention of erectile dysfunction, pulmonary arterial hypertension, chronic obstructive pulmonary disease, benign prostatic hypertrophy and lower urinary tract diseases, which comprises the above SK-3530 salt as an active ingredient.

Hereunder is given a more detailed description of the present invention.

The present invention provides the non-hygroscopic pharmaceutically acceptable fumarate salt of SK-3530 represented by the following formula (1), which has superior stabilities and medicinal effects. It also shows maximum blood concentration at a physiologically appropriate time and thus is useful for the treatment and prevention of erectile dysfunction, pulmonary arterial hypertension, chronic obstructive pulmonary disease, benign prostatic hypertrophy and lower urinary tract diseases:

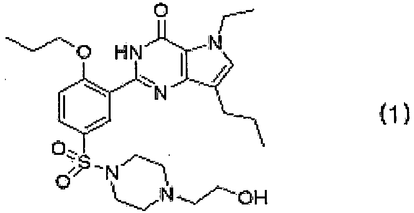

The present invention also provides a preparation process of the SK-3530 salt, which comprises the steps of reacting a free base of SK-3530 represented by the formula (1) with the pharmaceutically acceptable fumaric acid.

The process of preparing the crystalline acid addition salt of SK-3530 according to the present invention comprises:
dissolving or suspending
   fumaric acid to prepare an acid solution;
mixing the acid solution with a free base of SK-3530; and
filtering, washing and drying the solid obtained by stirring the above mixture to obtain a crystalline acid addition salt.

When preparing the mixture of a free base of SK-3530 and an acid in preparing the crystalline acid addition salt of SK-3530 according to the present invention, the pharmaceutically acceptable fumaric acid may be added to the free base of SK-3530 or the free base of SK-3530 may be added to the acid.

Hereunder is given a detailed description of each step of the preparation process according to the present invention.

In the first step of preparing the acid solution, the control of the concentration of the acid is important. Preferably, the concentration of the acid is controlled within 1 to 30 wt % in order to effectively promote crystallization.

In the second step of preparing the mixture of a free base of SK-3530 and the acid, the acid is preferably used in the amount of 0.5 to 3.0 equivalent ratio relative to SK-3530. When preparing the mixture, the acid may be added to the free base of SK-3530 or the free base of SK-3530 may be added to the acid. The free base of SK-3530 may be added in solid state or as dissolved in an appropriate reaction solvent. To describe in more detail, a free base of SK-3530 in solid state or dissolved in an appropriate solvent may be added to the acid solution to prepare the mixture. Alternatively, the acid solution may be added to a free base of SK-3530 in solid form or a free base of SK-3530 solution dissolving in an appropriate solvent.

In the second and third steps, water or a commonly used organic solvent is used as a reaction solvent. Particularly, it is preferable to use water or an organic solvent selected from acetone, methanol, ethanol, isopropanol and acetonitrile and a combination thereof.

In the third step, a crystalline acid addition salt is formed at from -30 to 50 °C, preferably from 0 to 30 °C, particularly preferably around room temperature of 15 to 25 °C.

The fumarate salt of SK-3530 represented by the formula (1) provided by the present invention satisfies all the following five physicochemical requirements required for a pharmaceutically acceptable salt - (1) low hygroscopicity, (2) adequate solubility, (3) less adhesiveness of tablet, (4) superior stability and (5) easiness of mass production.

Accordingly, the present invention comprises a pharmaceutical composition for treating erectile dysfunction which comprises the fumarate salt of the above SK-3530 represented by the formula (1) as an active ingredient.

The pharmaceutical composition according to the present invention can be administered orally or non-orally and can be made into common medicinal preparation forms. That is, it can be prepared into various medication forms for oral and non-oral administration. A commonly used diluent or excipient, including a filler, an extender, a binder, a wetting agent, a disintegrant, a surfactant, etc., is used for the preparation. Solid medication forms for oral administration include tablet, pill, powder, granule and capsule. These solid medication forms are prepared by mixing at least one excipient, for example, starch, sucrose or lactose, gelatin, and so forth, with the active ingredient. Further, in addition to simple excipients, lubricant such as magnesium stearate and talc is used. Liquid medication forms for oral administration include suspension, solution, emulsion and syrup. In addition to the commonly used diluent such as water and liquid paraffin, various excipients, for example, a wetting agent, a sweetener, a flavor, a preservative, etc., may be used Medication forms for non-oral administration include a sterilized aqueous solution, a non-aqueous solution, a suspension, an emulsion, a lyophilized medication and a suppository. For a non-aqueous solution and a suspension, propylene glycol, polyethylene glycol, plant oil like olive oil, injectable ester like ethyl oleate, etc., may be used. For the suppository base, Witepsol, Macrogol, Tween 61, cacao fat, laurin fat, glycerogelatin, etc., may be used.

The administration dosage of the pharmaceutical composition according to the present invention may vary depending on the patient's age, body weight, sex, administration route, physical conditions and severity of disease. Effective administration dosage of the SK-3530 salt is 10.0-200.0 mg, preferably 20-150 mg based on the weigh of the free base of SK-3530.

### [Best Mode for Carrying Out the Invention]

Practical and presently preferred embodiments of the present invention are illustrated in the following examples. However, it will be appreciated that those skilled in the art may, in consideration of this disclosure, make modifications and improvements within the spirit and scope of the present invention.

### Comparative Example 1: Preparation of SK-3530 gentisate salt

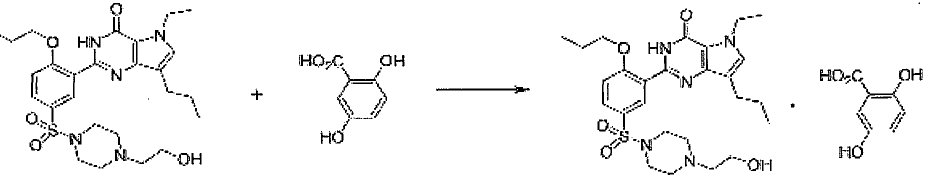

2.44 g of gentisic acid was dissolved in 100 mL of acetone and the resultant solution was stirred at room temperature. 8.0 g of a free base of SK-3530 was dissolved in 100 mL of acetone and slowly added to the gentisic acid solution. The mixture was stirred for 1 hour at room temperature and the resultant solid was filtered, washed with 20 mL of acetone and dried in vacuum at 50 °C to obtain 7.96 g (yield: 77.1 %) of a white crystalline target compound.
¹H-NMR (300MHz, DMSO-d₆) δ (ppm) 11.70 (s, 1H), 7.89 (d, 1H), 7.80 (d.d., 1 H), 7.38 (d, 1 H), 7.31 (s, 1 H), 7.14 (d, 1 H), 6.87 (d.d., 1 H), 6.71 (d, 1 H), 4.37 (q, 2H), 4.12 (t, 2H), 3.47 (t, 2H), 2.95 (m, 4H), 2.66 (m, 4H), 2.59∼2.48 (m, 4H), 1.77∼1.59 (m, 4H), 1.35 (t, 3H), 0.96 (t, 3H), 0.92 (t, 3H)

### Comparative Example 2: Preparation of SK-3530 gentisate salt

2.44 g of gentisic acid was dissolved in 100 mL of acetone and the resultant solution was stirred at room temperature. 8.0 g of a free base of SK-3530 was slowly added to the gentisic acid solution. The mixture was stirred for 1 hour at room temperature and the resultant solid was filtered, washed with 20 mL of acetone and dried in vacuum at 50 °C to obtain a white crystalline target compound.

### Comparative Example 3: Preparation of SK-3530 gentisate salt

200 mg of a free base of SK-3530 was suspended in 1 mL of acetone and the resultant solution was stirred at room temperature. 61 mg of gentisic acid was dissolved in a mixed solvent of acetone (1 mL) and water (2 mL) and slowly added to the solution of the free base of SK-3530. The mixture was stirred for 30 minutes at room temperature and further stirred for 30 minutes after adding 12 mL of water. The resultant solid was filtered, washed with 10 mL of water and dried in vacuum at 50 °C to obtain 249 mg (yield: 96.5 %) of a white crystalline target compound.

### Comparative Example 4: Preparation of tablet containing SK-3530 gentisate salt

Anhydrous dibasic calcium phosphate (315 g) was mixed with microcrystalline cellulose (525 g, 90 µm) and transferred into a drum. Subsequently, SK-3530 gentisate salt (70 g) was mixed with microcrystalline cellulose (187.5 g, 50 µm) and screened through into the drum containing the aforesaid powder mixture. The screen was cleaned with microcrystalline cellulose (525 g, 90 µm). Anhydrous dibasic calcium phosphate (315 g) was added to the mixture and blended for 10 minutes. Subsequently, sodium starch glycolate (40 g) was added to the mixture and blended for 6 minutes. Finally, magnesium stearate (20 g) was added and blended for 3 minutes. The resultant powder mixture was compacted into a tablet by the common method.

### Comparative Example 5: Preparation of capsule containing SK-3530 gentisate salt

Microcrystalline cellulose (525 g, 90 µm) was mixed with dry cornstarch. SK-3530 gentisate salt (70 g) was mixed with part of the premixture and screened through a sieve. The remaining cornstarch was added and, after 10 minutes of mixing, sieving was performed followed by 5 minutes of further mixing. The product was filled into a capsule of an appropriate size.

### Comparative Example 6: Preparation of injection containing SK-3530 gentisate salt

Sodium chloride was dissolved in sterile water for injection and mixed with propylene glycol. SK-3530 gentisate salt was added and, after dissolving, sterile water for injection was further added to obtain a solution with wanted concentration. The resultant solution was filtered through a sterilizing filter and filled into a sterilized ampule used for the container for injection.

### Comparative Example 7: Preparation of SK-3530 maleate salt

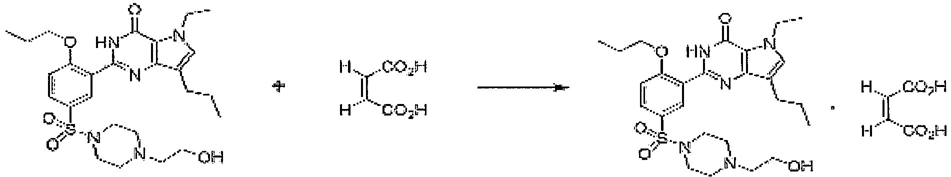

1.44 g of maleic acid was dissolved in 100 mL of acetone and the resultant solution was stirred at room temperature. 6.0 g of a free base of SK-3530 was slowly added to the maleic acid solution. The mixture was stirred for 1 hour at room temperature and 50 mL of acetone was removed by condensation under reduced pressure. The resultant solid was filtered, washed with 20 mL of ether and dried in vacuum at 50 °C to obtain 7.02 g (yield: 96.0 %) of a white crystalline target compound.
¹H-NMR (300MHz, DMSO-d₆) δ (ppm) 11.73 (s, 1H), 7.94 (d, 1H), 7.84 (d.d., 1H), 7.42 (d, 1 H), 7.32 (s, 1 H), 6.08 (s, 2H), 4.37 (q, 2H), 4.14 (t, 2H), 3.62 (t, 2H), 3.52-2.70 (m, 10H), 2.57 (t, 2H), 1.79-1.60 (m, 4H), 1.36 (t, 3H), 0.97 (t, 3H), 0.92 (t, 3H)

### Comparative Example 8: Preparation of SK-3530 maleate salt

1.44 g of maleic acid was dissolved in 100 mL of acetone and the resultant solution was stirred at room temperature. 6.0 g of a free base of SK-3530 was dissolved in 100 mL of acetone and slowly added to the maleic acid solution. The mixture was stirred for 1 hour at room temperature and 50 mL of acetone was removed by condensation under reduced pressure. The resultant solid was filtered, washed with 20 mL of ether and dried in vacuum at 50 °C to obtain a white crystalline target compound.

### Comparative Example 9: Preparation of SK-3530 maleate salt

60 mg of a free base of SK-3530 was suspended in 1 mL of acetone and the resultant solution was stirred at room temperature. 14.4 mg of maleic acid was dissolved in a mixed solvent of acetone (1 mL) and water (2 mL) and slowly added to the solution of the free base of SK-3530. The mixture was stirred for 1 hour at room temperature and the resultant solid was filtered, washed with 20 mL of acetone and dried in vacuum at 50 °C to obtain a white crystalline target compound.

### Comparative Example 10: Preparation of tablet containing SK-3530 maleate salt

Anhydrous dibasic calcium phosphate (315 g) was mixed with microcrystalline cellulose (525 g, 90 µm) and transferred into a drum. Subsequently, SK-3530 maleate salt (70 g) was mixed with microcrystalline cellulose (187.5 g, 50 µm) and screened through into the drum containing the aforesaid powder mixture. The screen was cleaned with microcrystalline cellulose (525 g, 90 µm). Anhydrous dibasic calcium phosphate (315 g) was added to the mixture and blended for 10 minutes. Subsequently, sodium starch glycolate (40 g) was added to the mixture and blended for 6 minutes. Finally, magnesium stearate (20 g) was added and blended for 3 minutes. The resultant powder mixture was compacted into a tablet by the common method.

### Comparative Example 11: Preparation of capsule containing SK-3530 maleate salt

Microcrystalline cellulose (525 g, 90 µm) was mixed with dry cornstarch. SK-3530 maleate salt (70 g) was mixed with part of the premixture and screened through a sieve. The remaining cornstarch was added and, after 10 minutes of mixing, sieving was performed followed by 5 minutes of further mixing. The product was filled into a capsule of an appropriate size.

### Comparative Example 12: Preparation of injection containing SK-3530 maleate salt

Sodium chloride was dissolved in sterile water for injection and mixed with propylene glycol. SK-3530 maleate salt was added and, after dissolving, sterile water for injection was further added to obtain a solution with wanted concentration. The resultant solution was filtered through a sterilizing filter and filled into a sterilized ampule used for the container for injection.

### Comparative Example 13: Preparation of SK-3530 citrate salt

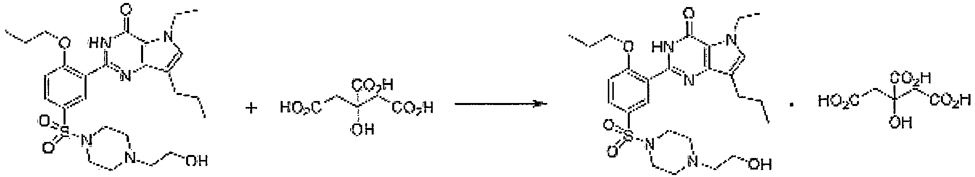

3.04 g of citric acid was dissolved in 100 mL of acetone and the resultant solution was stirred at room temperature. 8.0 g of a free base of SK-3530 was dissolved in 100 mL of acetone and slowly added to the citric acid solution. The mixture was stirred for 1 hour at room temperature and the resultant solid was filtered, washed with 20 mL of acetone and dried in vacuum at 50 °C to obtain 10.5 g (yield: 96.4 %) of a white crystalline target compound.
¹H-NMR (300MHz, DMSO-d₆) δ (ppm) 11.70 (s, 1H), 7.88 (d, 1 H), 7.80 (d.d., 1H), 7.38 (d, 1 H), 7.31 (s, 1 H), 4.37 (q, 2H), 4.12 (t, 2H), 3.44 (t, 2H), 3.00∼2.83 (m, 4H), 2.75∼2.54 (m, 8H), 2.51∼2.47 (m, 4H), 1.75∼1.62 (m, 4H), 1.35 (t, 3H), 0.96 (t, 3H), 0.92 (t,3H)

### Comparative Example 14: Preparation of SK-3530 citrate salt

3.04 g of citric acid was dissolved in 100 mL of acetone and the resultant solution was stirred at room temperature. 8.0 g of a free base of SK-3530 was dissolved in 100 mL of acetone and slowly added to the citric acid solution. The mixture was stirred for 1 hour at room temperature and the resultant solid was filtered, washed with 20 mL of acetone and dried in vacuum at 50 °C to obtain a white crystalline target compound.

### Comparative Example 15: Preparation of SK-3530 citrate salt

80 mg of a free base of SK-3530 was suspended in 1 mL of acetone and the resultant solution was stirred at room temperature. 30.4 mg of citric acid was dissolved in a mixed solvent of acetone (1 mL) and water (2 mL) and slowly added to the solution of the free base of SK-3530. The mixture was stirred for 30 minutes at room temperature and further stirred for 30 minutes after adding 12 mL of water. The resultant solid was filtered, washed with 10 mL of water and dried in vacuum at 50 °C to obtain a white crystalline target compound.

### Comparative Example 16: Preparation of tablet containing SK-3530 citrate salt

Anhydrous dibasic calcium phosphate (315 g) was mixed with microcrystalline cellulose (525 g, 90 µm) and transferred into a drum. Subsequently, SK-3530 citrate salt (70 g) was mixed with microcrystalline cellulose (187.5 g, 50 µm) and screened through into the drum containing the aforesaid powder mixture. The screen was cleaned with microcrystalline cellulose (525 g, 90 µm). Anhydrous dibasic calcium phosphate (315 g) was added to the mixture and blended for 10 minutes. Subsequently, sodium starch glycolate (40 g) was added to the mixture and blended for 6 minutes. Finally, magnesium stearate (20 g) was added and blended for 3 minutes. The resultant powder mixture was compacted into a tablet by the common method.

### Comparative Example 17: Preparation of capsule containing SK-3530 citrate salt

Microcrystalline cellulose (525 g, 90 µm) was mixed with dry cornstarch. SK-3530 citrate salt (70 g) was mixed with part of the premixture and screened through a sieve. The remaining cornstarch was added and, after 10 minutes of mixing, sieving was performed followed by 5 minutes of further mixing. The product was filled into a capsule of an appropriate size.

### Comparative Example 18: Preparation of injection containing SK-3530 citrate salt

Sodium chloride was dissolved in sterile water for injection and mixed with propylene glycol. SK-3530 citrate salt was added and, after dissolving, sterile water for injection was further added to obtain a solution with wanted concentration. The resultant solution was filtered through a sterilizing filter and filled into a sterilized ampule used for the container for injection.

### Inventive Example 19: Preparation of SK-3530 fumarate salt

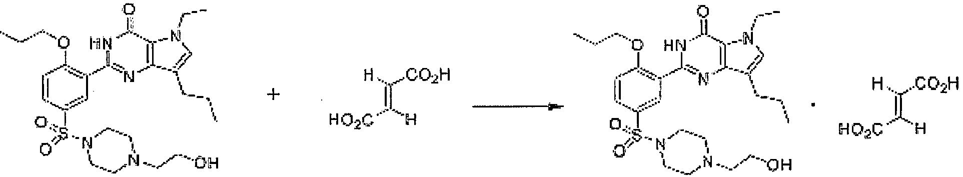

1.44 g of fumaric acid was dissolved in 100 mL of acetone and the resultant solution was stirred at room temperature. 6.0 g of a free base of SK-3530 was slowly added to the gentisic acid solution. The mixture was stirred for 1 hour at room temperature and, after removing 50 mL of acetone by condensation under reduced pressure, the resultant solid was filtered, washed with 20 mL of ether and dried in vacuum at 50 °C to obtain 6.92 g (yield: 94.7 %) of a white crystalline target compound.
¹H-NMR (300MHz, DMSO-d₆) δ (ppm) 11.69 (s, 1H), 7.88 (d, 1H), 7.79 (d.d., 1H), 7.37 (d, 1H), 7.30 (s, 1H), 6.62 (s, 2H), 4.37 (q, 2H), 4.12 (t, 2H), 3.43 (t, 2H), 2.90 (m, 4H), 2.59∼2.48 (m, 6H), 2.40 (t, 2H), 1.75∼1.59 (m, 4H), 1.35 (t, 3H), 0.96 (t, 3H), 0.92 (t, 3H)

### Inventive Example 20: Preparation of SK-3530 fumarate salt

1.44 g of fumaric acid was dissolved in 100 mL of acetone and the resultant solution was stirred at room temperature. 8.0 g of a free base of SK-3530 was dissolved in 100 mL of acetone and slowly added to the fumaric acid solution. The mixture was stirred for 1 hour at room temperature and the resultant solid was filtered, washed with 20 mL of acetone and dried in vacuum at 50 °C to obtain a white crystalline target compound.

### Inventive Example 21: Preparation of SK-3530 fumarate salt

80 mg of a free base of SK-3530 was suspended in 1 mL of acetone and the resultant solution was stirred at room temperature. 14.4 mg of fumaric acid was dissolved in a mixed solvent of acetone (1 mL) and water (2 mL) and slowly added to the solution of the free base of SK-3530. The mixture was stirred for 30 minutes at room temperature and further stirred for 30 minutes after adding 12 mL of water. The resultant solid was filtered, washed with 10 mL of water and dried in vacuum at 50 °C to obtain a white crystalline target compound.

### Inventive Example 22: Preparation of tablet containing SK-3530 fumarate salt

Anhydrous dibasic calcium phosphate (315 g) was mixed with microcrystalline cellulose (525 g, 90 µm) and transferred into a drum. Subsequently, SK-3530 fumarate salt (70 g) was mixed with microcrystalline cellulose (187.5 g, 50 µm) and screened through into the drum containing the aforesaid powder mixture. The screen was cleaned with microcrystalline cellulose (525 g, 90 µm). Anhydrous dibasic calcium phosphate (315 g) was added to the mixture and blended for 10 minutes. Subsequently, sodium starch glycolate (40 g) was added to the mixture and blended for 6 minutes. Finally, magnesium stearate (20 g) was added and blended for 3 minutes. The resultant powder mixture was compacted into a tablet by the common method.

### Inventive Example 23: Preparation of capsule containing SK-3530 fumarate salt

Microcrystalline cellulose (525 g, 90 µm) was mixed with dry cornstarch. SK-3530 fumarate salt (70 g) was mixed with part of the premixture and screened through a sieve. The remaining cornstarch was added and, after 10 minutes of mixing, sieving was performed followed by 5 minutes of further mixing. The product was filled into a capsule of an appropriate size.

### Inventive Example 24: Preparation of injection containing SK-3530 fumarate salt

Sodium chloride was dissolved in sterile water for injection and mixed with propylene glycol. SK-3530 fumarate salt was added and, after dissolving, sterile water for injection was further added to obtain a solution with wanted concentration. The resultant solution was filtered through a sterilizing filter and filled into a sterilized ampule used for the container for injection.

### Comparative Example 25: Preparation of SK-3530 hemitartrate salt

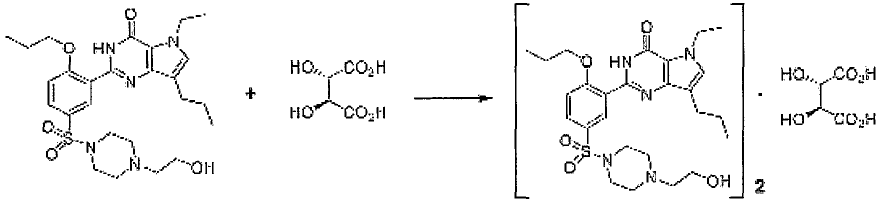

1.19 g of tartaric acid was dissolved in 100 mL of acetone and the resultant solution was stirred at room temperature. 8.0 g of a free base of SK-3530 was dissolved in 100 mL of acetone and slowly added to the tartaric acid solution. The mixture was stirred for 1 hour at room temperature and the resultant solid was filtered, washed with 20 mL of acetone and dried in vacuum at 50 °C to obtain 7.6 g (yield: 83.2 %) of a white crystalline target compound.
¹H-NMR (300MHz, DMSO-d₆) δ (ppm) 11.70 (s, 1H), 7.87 (d, 1H), 7.79 (d.d., 1H), 7.38 (d, 1 H), 7.31 (s, 1 H), 4.36 (q, 2H), 4.26 (s, 1 H), 4.12 (t, 2H), 3.42 (t, 2H), 2.89 (m, 4H), 2.59∼2.47 (m, 6H), 2.39 (t, 2H), 1.80∼1.56 (m, 4H), 1.35 (t, 3H), 0.96 (t, 3H), 0.92 (t, 3H)

### Comparative Example 26: Preparation of SK-3530 hemitartrate salt

1.19 g of tartaric acid was dissolved in 100 mL of acetone and the resultant solution was stirred at room temperature. 8.0 g of a free base of SK-3530 was slowly added to the tartaric acid solution. The mixture was stirred for 1 hour at room temperature and the resultant solid was filtered, washed with 20 mL of acetone and dried in vacuum at 50 °C to obtain a white crystalline target compound.

### Comparative Example 27: Preparation of tablet containing SK-3530 hemitartrate salt

Anhydrous dibasic calcium phosphate (315 g) was mixed with microcrystalline cellulose (525 g, 90 µm) and transferred into a drum. Subsequently, SK-3530 hemitartrate salt (70 g) was mixed with microcrystalline cellulose (187.5 g, 50 µm) and screened through into the drum containing the aforesaid powder mixture. The screen was cleaned with microcrystalline cellulose (525 g, 90 µm). Anhydrous dibasic calcium phosphate (315 g) was added to the mixture and blended for 10 minutes. Subsequently, sodium starch glycolate (40 g) was added to the mixture and blended for 6 minutes. Finally, magnesium stearate (20 g) was added and blended for 3 minutes. The resultant powder mixture was compacted into a tablet by the common method.

### Comparative Example 28: Preparation of capsule containing SK-3530 hemitartrate salt

Microcrystalline cellulose (525 g, 90 µm) was mixed with dry cornstarch. SK-3530 hemitartrate salt (70 g) was mixed with part of the premixture and screened through a sieve. The remaining cornstarch was added and, after 10 minutes of mixing, sieving was performed followed by 5 minutes of further mixing. The product was filled into a capsule of an appropriate size.

### Comparative Example 29: Preparation of injection containing SK-3530 hemitartrate salt

Sodium chloride was dissolved in sterile water for injection and mixed with propylene glycol. SK-3530 hemitartrate salt was added and, after dissolving, sterile water for injection was further added to obtain a solution with wanted concentration. The resultant solution was filtered through a sterilizing filter and filled into a sterilized ampule used for the container for injection.

### Testing Example 1: Stability test

This test is for confirming the storage stability of SK-3530 salt.

### 1) Water and atmospheric stability

A sufficient stability is required to process a drug into a particular medication form. For instance, preparation into tablet or capsule requires atmospheric stability and preparation into injection may require water stability.

The following Table 1 (25 °C, 75 % humidity), Table 2 (40 °C, 60 % humidity) and Table 3 (50 °C, 75 % humidity) show the content of total impurities measured by liquid chromatography after storing dihydrochloride (2HCl), gentisate, maleate, citrate, fumarate and hemitartrate salts of SK-3530 for 1 week and 3 weeks.

**[Table 1]**

| SK-3530 salts | Impurity content (%) at 25 °C, 75 % humidity | | |
|---|---|---|---|
| | Initial | 1 week | 3 weeks |
| SK-3530 dihydrochloride salt | 0.20 | 0.24 | 0.31 |
| SK-3530 gentisate salt | 0.09 | 0.09 | 0.12 |
| SK-3530 maleate salt | 0.12 | 0.12 | 0.12 |
| SK-3530 citrate salt | 0.12 | 0.15 | 0.15 |
| SK-3530 fumarate salt | 0.02 | 0.02 | 0.03 |
| SK-3530 hemitartrate salt | 0.07 | 0.09 | 0.13 |

**[Table 2]**

| SK-3530 salts | Impurity content (%) at 40 °C, 60 % humidity | | |
|---|---|---|---|
| | Initial | 1 week | 3 weeks |
| SK-3530 dihydrochloride salt | 0.20 | 0.24 | 0.38 |
| SK-3530 gentisate salt | 0.09 | 0.10 | 0.10 |
| SK-3530 maleate salt | 0.12 | 0.12 | 0.12 |
| SK-3530 citrate salt | 0.12 | 0.13 | 0.14 |
| SK-3530 fumarate salt | 0.02 | 0.02 | 0.05 |
| SK-3530 hemitartrate salt | 0.07 | 0.10 | 0.14 |

**[Table 3]**

| SK-3530 salts | Impurity content (%) at 50 °C, 75 % humidity | | |
|---|---|---|---|
| | Initial | 1 week | 3 weeks |
| SK-3530 dihydrochloride salt | 0.20 | 0.27 | 0.42 |
| SK-3530 gentisate salt | 0.09 | 0.09 | 0.10 |
| SK-3530 maleate salt | 0.12 | 0.15 | 0.19 |
| SK-3530 citrate salt | 0.12 | 0.14 | 0.14 |
| SK-3530 fumarate salt | 0.02 | 0.02 | 0.05 |
| SK-3530 hemitartrate salt | 0.07 | 0.11 | 0.13 |

### 2) Photostability test

The following Table 4 and Table 5 show the photostability test result for dihydrochloride salt (2HCl), gentisate, maleate, citrate, fumarate and hemitartrate salts of SK-3530. The total ultraviolet (UV) radiation was 200 W·h/m² and the total visible light radiation was 1080 klux/m²h. Each salt was kept on a Petri dish under the condition of 25 °C and 60 % humidity.

**[Table 4]**

| SK-3530 salts | Impurity content (%) | | |
|---|---|---|---|
| | Initial | UV | Visible |
| SK-3530 dihydrochloride salt | 0.20 | 5.92 | 1.37 |
| SK-3530 gentisate salt | 0.09 | 0.36 | 0.17 |
| SK-3530 maleate salt | 0.12 | 0.35 | 0.14 |
| SK-3530 citrate salt | 0.12 | 0.37 | 0.14 |
| SK-3530 fumarate salt | 0.02 | 0.08 | 0.07 |
| SK-3530 hemitartrate salt | 0.07 | 0.52 | 0.21 |

**[Table 5]**

| SK-3530 salts | Color change | | |
|---|---|---|---|
| | Initial | UV | Visible |
| SK-3530 dihydrochloride salt | White | Brown | Yellow |
| SK-3530 gentisate salt | White | Pale yellow | Pale yellow |
| SK-3530 maleate salt | White | White | White |
| SK-3530 citrate salt | White | White | White |
| SK-3530 fumarate salt | White | Pale yellow | White |
| SK-3530 hemitartrate salt | White | White | White |

### 3) Thermal stability test

The following Table 6 and Table 7 show the thermal stability test result for dihydrochloride salt (2HCl), gentisate, maleate, citrate, fumarate and hemitartrate salts of SK-3530. Each salt was placed on a Petri dish and kept in a dryer at 105 °C. Following macroscopic observation at 3 hours and 48 hours later, the content of impurities was measured with liquid chromatography.

**[Table 6]**

| SK-3530 salts | Impurity content (%) | | |
|---|---|---|---|
| | Initial | 3 hours | 48 hours |
| SK-3530 dihydrochloride salt | 0.20 | 3.06 | 14.37 |
| SK-3530 gentisate salt | 0.09 | 0.09 | 0.13 |
| SK-3530 maleate salt | 0.12 | 0.15 | 0.74 |
| SK-3530 citrate salt | 0.12 | 0.30 | 1.81 |
| SK-3530 fumarate salt | 0.02 | 0.11 | 0.61 |
| SK-3530 hemitartrate salt | 0.07 | 0.14 | 0.70 |

**[Table 7]**

| SK-3530 salts | Color change | | |
|---|---|---|---|
| | Initial | 3 hours | 48 hours |
| SK-3530 dihydrochloride salt | White | White | Yellow |
| SK-3530 gentisate salt | White | White | White |
| SK-3530 maleate salt | White | White | White |
| SK-3530 citrate salt | White | White | White |
| SK-3530 fumarate salt | White | White | White |
| SK-3530 hemitartrate salt | White | White | White |

As shown in Tables 1 to 7, the gentisate, maleate, citrate, fumarate or hemitartrate salt of SK-3530, only the fumarate salt being in accordance with the present invention showed much superior storage stability, photostability against UV and visible light and thermal stability, when compared with the SK-3530 dihydrochloride salt.

### [Industrial Applicability]

As described above, the gentisate, maleate, citrate, fumarate or hemitartrate salt of SK-3530, only the fumarate salt being in accordance with the present invention is a crystalline acid addition salt suitable for pharmaceutical preparation and, with superior PDE-5 inhibiting activity, can be used for the treatment and prevention of erectile dysfunction, pulmonary arterial hypertension, chronic obstructive pulmonary disease, benign prostatic hypertrophy and lower urinary tract diseases.

## Claims

1. An acid addition salt of a pyrrolopyrimidinone derivative represented by the following formula (1), 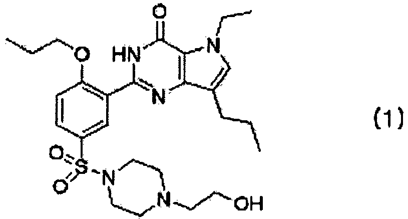 which is the fumarate salt.

2. A method of preparing an acid addition salt of a pyrrolopyrimidinone derivative by reacting a pyrrolopyrimidinone derivative represented by the following formula (1) 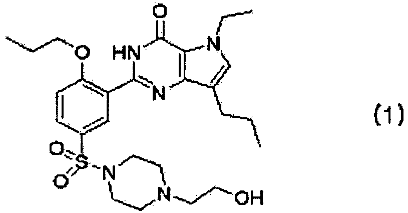 with fumaric acid.

3. The method according to claim 2, which comprises:
dissolving or suspending fumaric acid to prepare an acid solution;
mixing a pyrrolopyrimidinone derivative represented by the above formula (1) with the acid solution; and
filtering, washing and drying the solid obtained by stirring the above mixture to obtain a crystalline acid addition salt.

4. The method according to claim 3, wherein the acid concentration of the acid solution is 1 to 30 wt %.

5. The method according to claim 3 or claim 4, wherein the solvent used to dissolve or suspend the acid is a single solvent selected from the group consisting of water, acetone, methanol, ethanol, isopropanol and acetonitrile or a mixed solvent thereof.

6. The method according to claim 2 or claim 3, wherein the acid is used in the amount of 0.5 to 3.0 equivalent ratio relative to the pyrrolopyrimidinone derivative represented by the formula (1).

7. The method according to claim 2 or claim 3, wherein the reaction is performed at -30 to 50 °C.

8. The method according to claim 2 or 3, wherein the pyrrolopyrimidinone derivative represented by the formula (1) is used in solid state or as dissolved in a solvent.

9. The method according to claim 8, wherein the solvent used to dissolve the pyrrolopyrimidinone derivative represented by the formula (1) is a single solvent selected from the group consisting of water, acetone, methanol, ethanol, isopropanol and acetonitrile or a mixed solvent thereof.

10. A pharmaceutical composition for the treatment and prevention of erectile dysfunction comprising an acid addition salt of a pyrrolopyrimidinone derivative represented by the following formula (1), which is the fumarate salt, as an active ingredient:

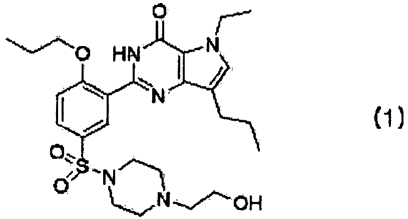

11. A pharmaceutical composition for the treatment and prevention of pulmonary arterial hypertension, chronic obstructive pulmonary disease, benign prostatic hypertrophy and lower urinary tract diseases comprising an acid addition salt of a pyrrolopyrimidinone derivative represented by the following formula (1), 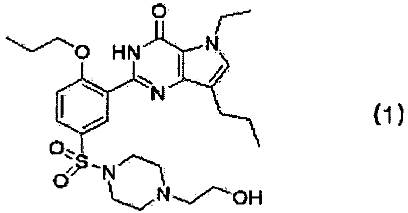 which is the fumarate salt, as an active ingredient.

12. The pharmaceutical composition as set forth in claim 10 or claim 11, which is in prepared in the form of a tablet, a capsule or an injection.

## Patentansprüche

1. Säureadditionssalz eines durch die folgende Formel (1) 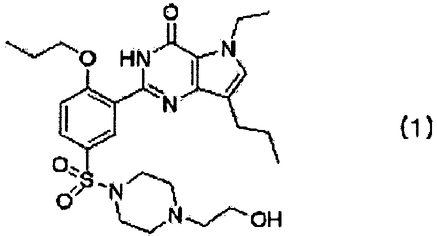 repräsentierten Pyrrolpyrimidinonderivats, welches das Fumaratsalz ist.

2. Verfahren zum Herstellen eines Säureadditionssalzes eines Pyrrolpyrimidinonderivats durch Umsetzen eines Pyrrolpyrimidinonderivats, welches durch die folgende Formel (1) 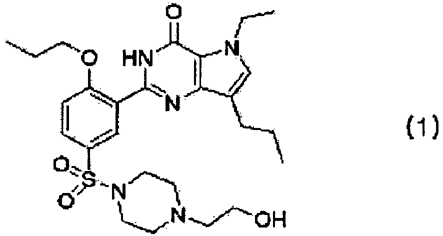 repräsentiert wird, mit Fumarsäure.

3. Verfahren nach Anspruch 2, umfassend die Verfahrensschritte:
Lösen oder Suspendieren von Fumarsäure, um eine Säurelösung herzustellen,
Mischen eines Pyrrolpyrimidinonderivats, welches durch die obige Formel (1) dargestellt ist, mit der Säurelösung, und
Filtern, Waschen und Trocknen des durch Rühren des obigen Gemischs erhaltenen Feststoffs, um ein kristallines Säureadditionssalz zu erhalten.

4. Verfahren nach Anspruch 3, wobei die Säurekonzentration der Säurelösung 1 bis 30 Gew.-% beträgt.

5. Verfahren nach Anspruch 3 oder Anspruch 4, wobei das zum Lösen oder Suspendieren der Säure verwendete Lösungsmittel ein einzelnes Lösungsmittel ist, das aus der Gruppe bestehend aus Wasser, Aceton, Methanol, Ethanol, Isopropanol und Acetonitril ausgewählt ist, oder ein Lösungsmittelgemisch davon ist.

6. Verfahren nach Anspruch 2 oder Anspruch 3, wobei die Säure in einem Äquivalentverhältnis in einer Menge von 0,5 bis 3,0 bezogen auf das Pyrrolpyrimidinonderivat, welches durch die Formel (1) repräsentiert wird, verwendet wird.

7. Verfahren nach Anspruch 2 oder Anspruch 3, wobei die Reaktion bei -30 bis 50 °C durchgeführt wird.

8. Verfahren nach Anspruch 2 oder 3, wobei das Pyrrolpyrimidinonderivat, welches durch die Formel (1) repräsentiert wird, im festen Zustand oder in einem Lösungsmittel gelöst eingesetzt wird.

9. Verfahren nach Anspruch 8, wobei das Lösungsmittel, welches zum Lösen des durch die Formel (1) dargestellten Pyrrolpyrimidinonderivats verwendet wird, ein einzelnes Lösungsmittel ist, das aus der Gruppe bestehend aus Wasser, Aceton, Methanol, Ethanol, Isopropanol und Acetonitril ausgewählt ist, oder ein Lösungsmittelgemisch davon ist.

10. Pharmazeutische Zusammensetzung zur Behandlung und Vorbeugung von Erektionsstörungen, welche als einen aktiven Bestandteil ein Säureadditionssalz eines durch die folgende Formel (1) 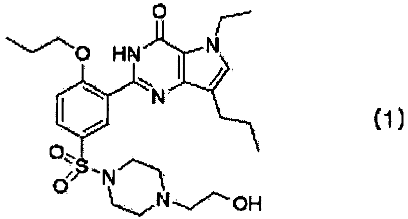 repräsentierten Pyrrolpyrimidinonderivats enthält, welches das Fumaratsalz ist.

11. Pharmazeutische Zusammensetzung zur Behandlung und Vorbeugung von pulmonaler arterieller Hypertonie, chronischer Lungenerkrankung, benigner Prostatahyperplasie und Erkrankungen der unteren Harnwege, welche als einen aktiven Bestandteil ein Säureadditionssalz eines durch die folgende Formel (1) 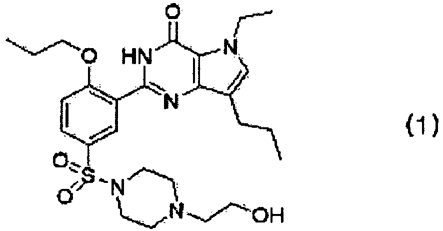 repräsentierten Pyrrolpyrimidinonderivats enthält, welches das Fumaratsalz ist.

12. Pharmazeutische Zusammensetzung nach Anspruch 10 oder Anspruch 11, welche in Form einer Tablette, einer Kapsel oder einer Injektion vorliegt.

## Revendications

1. Sel d'addition d'acide d'un dérivé de pyrrolopyrimidinone représenté par la formule (1) suivante : 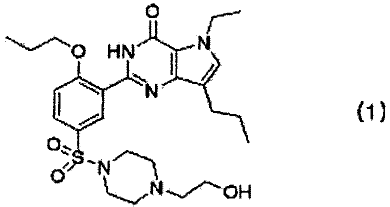 qui est le sel fumarate.

2. Procédé de préparation d'un sel d'addition d'acide d'un dérivé de pyrrolopyrimidinone par réaction d'un dérivé de pyrrolopyrimidinone représenté par la formule (1) suivante : 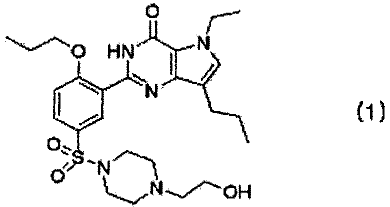 avec de l'acide fumarique.

3. Procédé selon la revendication 2, qui comprend :
la dissolution ou la mise en suspension de l'acide fumarique pour préparer une solution d'acide ;
le mélange d'un dérivé de pyrrolopyrimidinone représenté par la formule (1) ci-dessus avec la solution d'acide ; et
la filtration, le lavage et le séchage du solide obtenu par agitation du mélange ci-dessus pour obtenir un sel d'addition d'acide cristallin.

4. Procédé selon la revendication 3, dans lequel la concentration en acide de la solution d'acide est de 1 à 30% en poids.

5. Procédé selon la revendication 3 ou la revendication 4, dans lequel le solvant utilisé pour dissoudre ou mettre en suspension l'acide est un solvant unique choisi dans le groupe constitué par l'eau, l'acétone, le méthanol, l'éthanol, l'isopropanol et l'acétonitrile ou un solvant mixte constitué de ceux-ci.

6. Procédé selon la revendication 2 ou la revendication 3, dans lequel l'acide est utilisé à un rapport équivalent de 0,5 à 3,0 par rapport au dérivé de pyrrolopyrimidinone représenté par la formule (1).

7. Procédé selon la revendication 2 ou la revendication 3, dans lequel la réaction est réalisée à une température de -30 à 50°C.

8. Procédé selon la revendication 2 ou 3, dans lequel le dérivé de pyrrolopyrimidinone représenté par la formule (1) est utilisé à l'état solide ou sous forme dissoute dans un solvant.

9. Procédé selon la revendication 8, dans lequel le solvant utilisé pour dissoudre le dérivé de pyrrolopyrimidinone représenté par la formule (1) est un solvant unique choisi dans le groupe constitué par l'eau, l'acétone, le méthanol, l'éthanol, l'isopropanol et l'acétonitrile ou un solvant mixte constitué de ceux-ci.

10. Composition pharmaceutique destinée au traitement et à la prévention de la dysfonction érectile, comprenant un sel d'addition d'acide d'un dérivé de pyrrolopyrimidinone représenté par la formule (1) suivante, qui est le sel fumarate, en tant que principe actif :

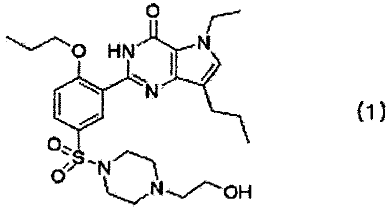

11. Composition pharmaceutique destinée au traitement et à la prévention de l'hypertension artérielle pulmonaire, de la bronchopneumopathie chronique obstructive, de l'adénome prostatique et des maladies du bas appareil urinaire, comprenant un sel d'addition d'acide d'un dérivé de pyrrolopyrimidinone représenté par la formule (1) suivante : 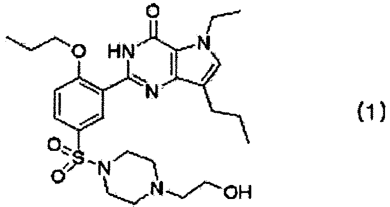 qui est le sel fumarate, en tant que principe actif.

12. Composition pharmaceutique telle qu'indiquée dans la revendication 10 ou la revendication 11, qui est préparée sous forme de comprimé, de capsule ou d'injection.
